# FASCICULE DE BREVET EUROPEEN

(11) **EP 1 563 304 B1**
(45) Date de publication et mention de la délivrance du brevet: **15.09.2010**
(21) Numéro de dépôt: 03786063.2
(22) Date de dépôt: 20.11.2003
(51) Int. Cl.: G01N 33/543, C08G 61/12

(54) **PROCEDE DE FIXATION D UNE PROTEINE SUR UN POLYMERE A BASE DE PYRROLE ET SON UTILISATION POUR LA FABRICATION D UN CAPTEUR**
VERFAHREN ZUR FIXIERUNG EINES PROTEINS AUF EINEM PYRROLPOLYMERISAT, UND DESSEN VERWENDUNG ZUR HERSTELLUNG EINES SENSORS
METHOD FOR FIXING A PROTEIN ON A PYRROLE-BASED POLYMER AND USE THEREOF FOR MAKING A SENSOR

(30) Priorité: 21.11.2002 FR 0214580
(43) Date de publication de la demande: 17.08.2005
(73) Titulaire: Commissariat à l'Énergie Atomique et aux Énergies Alternatives, 75015 Paris (FR); Centre National de la Recherche Scientifique, 75794 Paris Cedex 16 (FR)
(72) Inventeur: ROGET, André, F-38320 Brie et Angonnes (FR); LIVACHE, Thierry, F-38560 Haute-Jarrie (FR); LEVY, Yves, F-92240 Malakoff (FR)
(74) Mandataire: Poulin, Gérard
(86) Numéro de dépôt international: PCT/FR2003/050127
(87) Numéro de publication internationale: WO 2004/048972

(56) Documents cités:
- WO-A-00/36145
- FR-A- 2 750 136
- LIVACHE T ET AL: "Electroconducting polymers for the construction of DNA or peptide arrays on silicon chips." BIOSENSORS & BIOELECTRONICS. ENGLAND 15 SEP 1998, vol. 13, no. 6, 15 septembre 1998 (1998-09-15), pages 629-634, XP002249768 ISSN: 0956-5663
- BIDAN G ET AL: "Conducting polymers as a link between biomolecules and microelectronics" SYNTHETIC METALS, ELSEVIER SEQUOIA, LAUSANNE, CH, vol. 102, 1999, pages 1363-1365, XP002114817 ISSN: 0379-6779
- LIVACHE T ET AL: "Polypyrrole DNA chip on a silicon device: Example of hepatitis C virus typing" ANALYTICAL BIOCHEMISTRY, ACADEMIC PRESS, SAN DIEGO, CA, US, vol. 255, 1998, pages 188-194, XP002114813 ISSN: 0003-2697
- WOLOWACZ S E ET AL: "COVALENT ELECTROPOLYMERIZATION OF GLUCOSE OXIDASE IN POLYPYRROLE" ANALYTICAL CHEMISTRY, AMERICAN CHEMICAL SOCIETY. COLUMBUS, US, vol. 64, no. 14, 15 juillet 1992 (1992-07-15), pages 1541-1545, XP002030300 ISSN: 0003-2700
- YON-HIN B F Y ET AL: "COVALENT ELECTROPOLYMERIZATION OF GLUCOSE OXIDASE IN POLYPYRROLE. EVALUATION OF METHODS OF PYRROLE ATTACHMENT TO GLUCOSE OXIDASE ON THE PERFORMANCE OF ELCTROPOLYMERIZED GLUCOSE SENSORS" ANALYTICAL CHEMISTRY, AMERICAN CHEMICAL SOCIETY. COLUMBUS, US, vol. 65, 1993, pages 2067-2071, XP000885233 ISSN: 0003-2700

## Description

### DOMAINE TECHNIQUE

La présente invention se rapporte à un procédé de fixation d'une protéine sur un polymère à base de pyrrole, ainsi qu'à l'utilisation de ce procédé pour la fabrication de capteurs, en particulier de multicapteurs, par exemple de biopuces.

Les techniques de l'art antérieur n'ont pas été en mesure, ou très difficilement, d'immobiliser des protéines sur un polymère conducteur sans modifier leur activité, en particulier d'immobiliser deux protéines différentes séparément, notamment pour la fabrication de capteurs, notamment dans le domaine des biopuces.

En effet, les protéines, par exemple les enzymes, les anticorps, les récepteurs et certains antigènes, possèdent des sites de reconnaissance de molécules cibles ou des sites leur permettant d'être reconnues par d'autres molécules. Ces sites sont à l'origine de leur(s) activité(s). Par exemple, un enzyme possède un site spécifique pour son substrat, un anticorps possède un épitope spécifique permettant d'être reconnu par son antigène correspondant, et un antigène possède un site de reconnaissance spécifique de l'épitope qu'il cible. Ces sites sont généralement formés grâce à un repliement de la protéine sur elle même dans l'espace, repliement qui peut être stabilisé par des liaisons disulfure, ionique, hydrogène et hydrophobe. Ces repliements, et de fait les sites qu'ils forment, peuvent donc facilement être déstabilisés par une simple contrainte appliquée sur la protéine, par un encombrement stérique, par un changement de force ionique, de pH ou de température du milieu. En outre, ces sites présentent des fonctions réactives telles que des fonctions -NH₂, -OH, -COOH, -SH qui sont susceptibles de réagir avec les réactifs ou les monomères utilisés lors de la fixation de la protéines sur le polymère conducteur, et peuvent donc être endommagés, bloqués ou encombrés.

Les procédés utilisés pour fixer une protéine possédant un tel site sur une surface afin d'exploiter ce site doivent donc dans la mesure du possible préserver ce site de manière à ce que le capteur fabriqué puisse remplir sa fonction en fournissant des résultats d'analyse d'échantillon aussi précis, sensibles et reproductibles que possible.

### ETAT DE LA TECHNIQUE ANTERIEURE

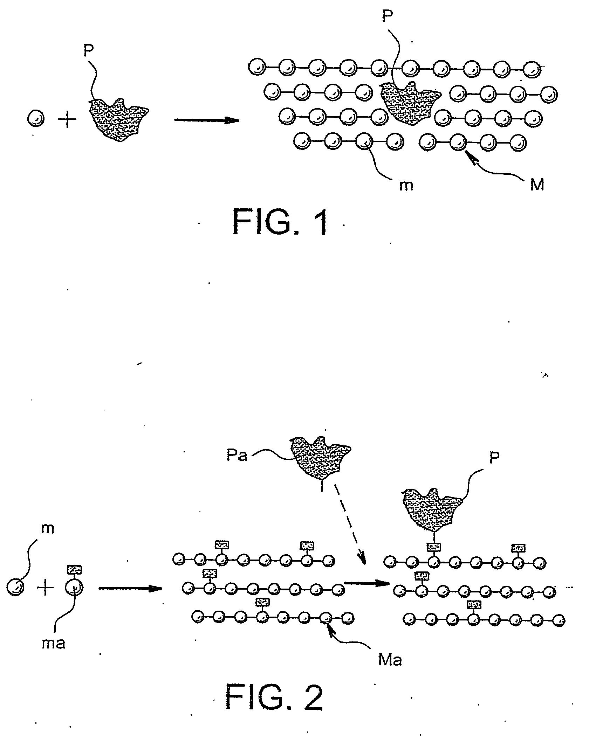

Dans les techniques connues de l'homme du métier, l'immobilisation de protéines sur un polymère conducteur peut faire appel à quatre techniques différentes. Ces quatre techniques sont l'emprisonnement ("entrapment" en anglais), la post-fonctionnalisation, l'immobilisation non covalente des protéines, et l'immobilisation covalente des protéines. Ces techniques sont représentées schématiquement sur les figures 1 à 4 annexées. Sur ces figures, « P » et « Pa » représentent respectivement la protéine et la protéine activée, « m » et « ma » représentent respectivement un monomère et un monomère activé du polymère, « P-m » représente un composé de couplage protéine monomère, et « M » et « Ma » représentent respectivement le polymère et le polymère activé.

Dans la technique de l'emprisonnement, le monomère est soumis à une polymérisation en présence de la protéine. Le polymère en se formant piège cette protéine dans son réseau. Cette technique, est décrite par exemple par Foulds et Lowe dans J. Chem. Soc., Faraday Trans. 1986, 82,1259-1264, et est illustrée sur la figure 1 annexée. Elle est intéressante car elle permet de ne pas modifier la protéine avant son immobilisation et donc, en principe, de mieux conserver son activité. Ce procédé a été repris plus récemment par OA Sadik et al., Talanta 2001, 55 : 929-941.

Malheureusement, ce procédé ne permet pas d'adresser par le courant uniquement la protéine sur une électrode choisie, il ne permet pas non plus de faire plusieurs dépôts de protéines différentes sans contamination des dépôts d'un dépôt à l'autre du fait du mécanisme d'immobilisation par emprisonnement. En effet, dans cette technique, des protéines peuvent être emprisonnées partiellement et relarguées lors de la polymérisation des dépôts suivants et donc contaminer ces derniers. En outre, le temps d'immobilisation est assez long et des contaminations sont possibles entre les dépôts ce qui rend difficile la fabrication de multicapteurs, et de ce fait est utilisé pour la fabrication de monocapteurs.

Par ailleurs, il s'agit d'une technique d'immobilisation dans la masse du polymère ce qui conduit aux différents inconvénients suivants :
- une diminution voire une suppression de l'accès au site actif de la protéine,
- du fait que la protéine est encagée dans un milieu de polarité très différente de celle du milieu étudié, par exemple un échantillon à analyser, la reconnaissance entre la protéine et sa cible est perturbée en plus de l'encombrement stérique précité,
- si la reconnaissance de la protéine et de sa cible nécessite ou induit un changement dans la structure de la protéine ce changement peut être gêné par la rigidité du polymère, et
- la détection de l'interaction de la protéine avec sa cible ne pourra se faire que dans la masse ce qui interdit les méthodes optiques d'analyse les plus courantes. En revanche, il est bien adapté à une détection électrochimique, par exemple sous la forme de biocapteurs.

Cette technique nécessite, d'autre part, une épaisseur de polymère assez importante pour encager la protéine. Ceci présente les inconvénients suivants :
- la quantité de protéine utilisée doit être plus importante qu'un dépôt simple, et
- l'exposition au courant électrique doit être plus longue ; d'après le document Sadik et al. cité ci-dessus, la densité de charge est de l'ordre de 2nC/mm² (soit une épaisseur de polymère de 500nm environ).

Un autre inconvénient encore est que si l'emprisonnement de la protéine n'est pas parfait, cela conduit à une perte de la quantité de protéine lors des polymérisations ou des utilisations successives ou lors du stockage avec une diminution de sensibilité du capteur et une contamination dans le cas où on aurait un multicapteur.

La technique de la post-fonctionnalisation est schématisée sur la figure 2 annexée. Il s'agit de greffage de protéines activées, soit par des esters activés, soit par des maléimides principalement, sur des supports fonctionnalisés par des NH₂ ou des SH ou, à l'inverse, de protéines fonctionnalisées ou non par l'utilisation des NH₂ des lysines ou des SH des cystéines de ces protéines, sur des supports activés par des esters activés ou maléimides respectivement. Cette méthode est décrite par exemple par W. Schuhman dans Mikrochim. Acta 121, 1-29 (1995). Elle permet un greffage en surface ce qui induit une détection de surface.

Ce greffage n'est malheureusement pas évident à contrôler du fait de la faible réactivité des fonctions mises en jeu. De plus, il est adapté à la réalisation de monocapteurs, et ne permet que très difficilement de fabriquer des multicapteurs. En effet, si l'on cherche à réaliser un multicapteur comportant plusieurs spots, la réactivité étant faible dans cette technique, par exemple toutes les fonctions du premier spot n'ont pas réagi quand on fixe la deuxième protéine sur le deuxième spot, donc il y a contamination de la deuxième protéine sur le premier spot. Pour éviter cela, il faut apporter la protéine uniquement sur le dépôt choisi. Ceci peut difficilement être miniaturisé car s'il est effectivement possible de déposer des microgouttes de façon localisée, leur vitesse d'évaporation est très rapide et la réaction est difficile à contrôler. Dans ce cas, il n'y a aucun intérêt à utiliser un support de type polymère conducteur car l'adressage doit être effectué de manière mécanique, par exemple par un système jet d'encre. De telles technologies sont utilisées pour fabriquer des puces à protéine sur lame de verre comme cela est décrit dans G. MacBeath Science, 08 septembre 2000, Vol. 289, 1760-1763.

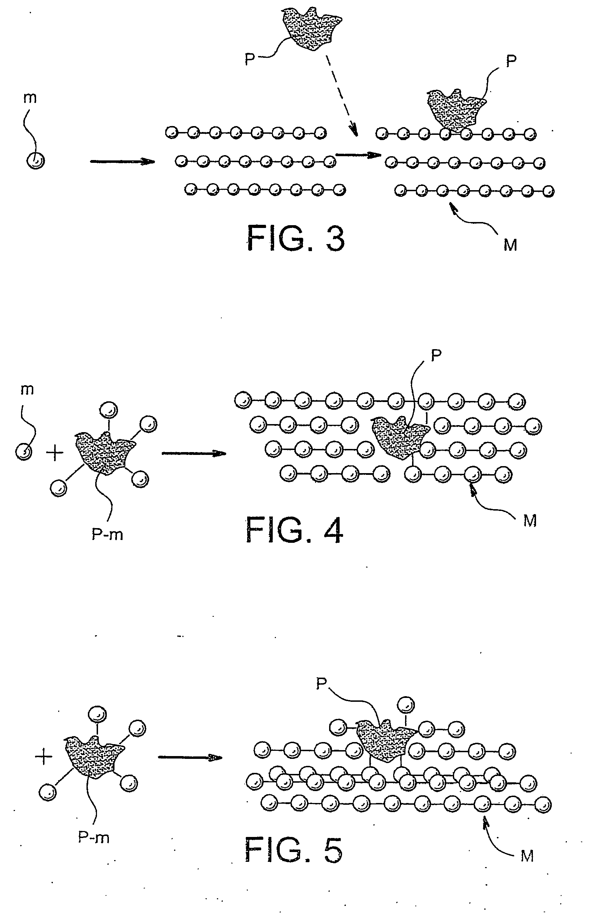

La technique d'immobilisation non covalente des protéines sans modification chimique préalable de ces protéines est représentée sur la figure 3 annexée. Dans cette technique, le système biotine-avidine est utilisé. Le document Farmakovsky et al WO 00/11473 décrit cette technique. Il y a formation d'un polymère conducteur avec des protéines plus ou moins bien immobilisées par adsorption.

Le fait que la liaison avec le support soit aussi faible n'est certainement pas favorable pour le stockage de cette électrode et encore moins à la fabrication d'un capteur multiple.

La quatrième technique est l'emprisonnement covalent. Elle est représentée schématiquement sur la figure 4 annexée. Cette technique a été décrite dans l'art antérieur par Wolowacz S.E., Yon Hin B.F. Y. et Lowe C.R. Anal. Chem.1992, 64,1541-1545. L'auteur a préparé une enzyme, la glucose oxydase, de manière à la conjuguer à du pyrrole et l'a soumise à une copolymérisation avec du pyrrole. Le pyrrole greffé sur l'enzyme électropolymérise et immobilise l'enzyme dans une sorte de gangue de polypyrrole.

Malheureusement l'enzyme est emprisonnée dans le pyrrole ce qui limite son accessibilité vis-à-vis du milieu extérieur et l'enfouissement de son site actif. Cette accessibilité peut dans certains cas être conservée mais seulement pour de très petites molécules, telles que le glucose dans la publication citée ci-dessus, susceptibles de pénétrer dans les mailles du réseau de polypyrrole. En fait, on se retrouve avec les inconvénients précités de la technique de l'emprisonnement citée ci-dessus, seule la stabilité est accrue.

Il existe donc un réel besoin pour un procédé de fixation d'une protéine sur un polymère conducteur qui ne présente pas les inconvénients, limitations, défauts et désavantages des procédés de l'art antérieur.

En outre, ce procédé doit être utilisable pour la fabrication d'un capteur, en particulier pour la fabrication d'un multicapteur, de manière à ce qu'il ne présente pas les inconvénients, limitations, défauts et désavantages des procédés et des capteurs de l'art antérieur.

### EXPOSÉ DE L'INVENTION

Le but de la présente invention est précisément de fournir un procédé de fixation d'une protéine sur un polymère conducteur, utilisable notamment pour la fabrication d'un monocapteur ou d'un multicapteur, qui réponde, entre autres, aux besoins indiqués ci-dessus.

Ce but, et d'autres encore, sont atteints, conformément à l'invention, par un procédé de fixation d'une protéine sur un support conducteur au moyen d'un polymère du pyrrole comprenant les étapes suivantes :
- couplage de la protéine à fixer avec un monomère du pyrrole de manière à obtenir une première solution d'un composé de couplage protéine-pyrrole,
- préparation d'une deuxième solution du monomère du pyrrole non couplé à la protéine,
- mélange de ladite première solution avec ladite deuxième solution pour obtenir une solution d'électropolymérisation,
- électropolymérisation du pyrrole et de la protéine couplée au pyrrole sur au moins une zone déterminée du support conducteur à partir de ladite solution d'électropolymérisation, ladite électropolymérisation étant réalisée en délivrant sur ladite zone une quantité de courant pous obtenir un polymère ayant une épaisseur égale à 10 nm au plus.

Selon l'invention, par monomère du pyrrole, on entend un monomère du pyrrole ou un dérivé polymérisable du pyrrole. Les monomères du pyrrole utilisables dans la présente invention sont ceux connu de l'homme du métier dans les procédés de fabrication des biopuces. En tant que dérivé du pyrrole, on peut citer par exemple, le méthyle pyrrole, ou des dimères de pyrrole subsitué ou non substitué.

Selon l'invention, une ou plusieurs protéines peuvent être fixées sur un support au moyen du procédé de l'invention. L'homme du métier saura, à la lumière de la présente description, préparer la solution d'électropolymérisation suivant ses besoins, avec une ou plusieurs protéines couplées chacune au monomère du pyrrole choisi, déposées chacune sur une ou plusieurs zone du support.

Selon l'invention, par zone du support, on entend aussi « plot », par exemple un plot d'un support de biopuce. Selon l'invention, l'électropolymérisation peut être réalisée simultanément sur plusieurs zones ou plots de la biopuce fabriquée, ou successivement sur plusieurs zones ou plots de la biopuce fabriquée, par exemple avec différentes protéines. Le choix du nombre de zones ou plots dépendra notamment de la résolution du biocapteur souhaitée, et du nombre d'analyses simultanées pouvant être effectuées avec le biocapteur souhaité. Le nombre de zones ou plots peut être déterminé comme pour les biopuces connues de l'homme du métier. Le support conducteur au sens de la présente invention peut donc être avantageusement un support de biopuce à un ou plusieurs plot(s). Le procédé de la présente invention permet donc de fabriquer au choix des monocapteurs ou des multicapteurs.

La présente invention permet de manière surprenante de conserver l'activité de la protéine fixée ou immobilisée sur le polymère, par exemple les propriétés de reconnaissance d'une protéine vis-à-vis de grosses molécules, par exemple d'un antigène vis-à-vis de ses anticorps correspondants.

Ceci n'était pas possible avec les techniques de l'art antérieur puisque le pyrrole réagissait sur la plupart des fonctions amines des protéines. Or, par le procédé de la présente invention, de manière tout à fait inattendue, les inventeurs ont généré des dépôts de polypyrrole-protéine dans lesquels l'activité de la protéine, immobilisé e dans ou sur une fine couche de polymère, est conservée. En outre, La présente invention permet de construire un film extrêmement fin, qui préserve d'avantage l'activité de la protéine.

En fait, ils ont préparé différents pyrroles qu'ils ont fait réagir sur des protéines comme cela est illustré dans les exemples ci-dessous. Ces protéines conjuguées sont ensuite immobilisées par électropolymérisation avec du pyrrole. Quoi qu'il en soit et malgré la méconnaissance du phénomène en cause, ils ont pu, par cette méthode, adresser sur un substrat conducteur des protéines antigéniques et les faire reconnaître par un anticorps spécifique.

Les inventeurs ont étudié cette reconnaissance par fluorescence ce qui leur a permis de vérifier que la protéine est bien immobilisée en surface puisque la fluorescence ne peut être détectée qu'en dehors de la matrice de polypyrrole. Ceci a permis de valider l'immobilisation par électro-copolymérisation conforme à la présente invention ainsi que la reconnaissance par une autre molécule qui peut être aussi encombrante qu'un anticorps.

Les inventeurs ont aussi réalisé une étude par imagerie de résonance plasmonique de surface (SPR) permettant d'aller plus loin en terme d'analyse par le fait que c'est une méthode d'analyse en temps réel. Ces études cinétiques permettent de mieux évaluer la conservation de l'activité grâce au procédé de la présente invention. Par ailleurs, cette méthode d'analyse est bien connue pour ne détecter que les interactions biologiques se déroulant à une très faible distance de la couche d'or comme cela est décrit dans P. GUEDON et al. Anal. Chem. (2000), 72, (24), 6003-6009. Cette distance est souvent inférieure à 50 nm.

Il découle de ces deux expériences que l'interaction se déroule à la fois à la surface du polymère (fluorescence) et très proche de l'or. Cela signifie que le polymère doit être extrêmement fin, ce qui est le cas grâce au procédé de l'invention.

Ainsi, selon l'invention, l'électropolymérisation est réalisée en limitant la quantité de courant délivrée lors de la polymérisation. En effet, de meilleurs résultats ont ainsi été obtenus. Il est possible que la quantité de courant conforme à la présente invention limite la longueur des chaînes polypyrrole. Ceci limiterait donc la perte d'activité de la protéine lors de son immobilisation. Une autre possibilité pourrait être que la polymérisation se passe au voisinage immédiat de l'électrode ce qui aurait pour effet de minimiser la polymérisation du pyrrole sur la partie de la protéine la plus éloignée de cette électrode et donc de conserver son activité de reconnaissance. Plus probablement, les inventeurs pensent qu'il s'agit d'un écrantage par la protéine moins conductrice que le milieu réactionnel vis-à-vis du champ électrique.

Ainsi, l'électropolymérisation est réalisée au moyen du procédé d'électropolymérisation électrochimique de la présente invention, en délivrant sur ladite zone une quantité de courant pouvant être délivrée par polarisation électrique de ladite zone qui permet la formation d'un polymère ayant une épaisseur pouvant être inférieure à 10 nm. On peut à titre d'exemple conduire cette polymérisation selon l'invention au moyen d'une impulsion électrique, appliquée à ladite zone, de 1 V/ECS durant 500 ms.

Selon l'invention, le couplage de la protéine à fixer avec du pyrrole peut être réalisé par activation du pyrrole suivi d'un couplage du pyrrole activé à la protéine à fixer. Le pyrrole activé peut par exemple être un ester activé du pyrrole qui est réactif sur les fonctions amines des lysines ou un maléimide pyrrole qui est réactif sur les fonctions -SH des cystéines. Ces exemples ne sont pas limitatifs, et l'homme du métier saura adapter le procédé de l'invention avec d'autres activations du pyrrole.

Selon l'invention, l'activation du pyrrole peut être réalisée par exemple au moyen de N-hydroxy-sulfosuccinimide ou de maléimide.

Selon l'invention, le composé de couplage protéine-pyrrole peut être avantageusement choisi parmi les composés suivants : 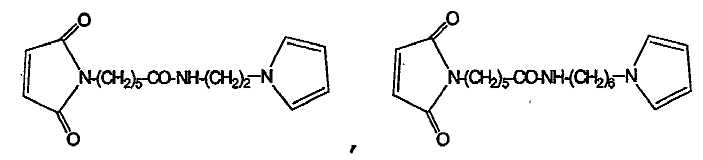 et

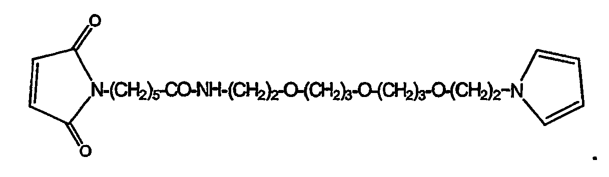

Selon un mode particulier de réalisation de la présente invention, plusieurs protéines peuvent être fixées sur le polymère du pyrrole, successivement et sur des zones différentes et déterminées du support conducteur. Par exemple, deux protéines peuvent être fixées sur le polymère du pyrrole, successivement et sur deux zones différentes déterminées du support conducteur.

Ce mode particulier de réalisation de l'invention consiste en d'autres termes en un procédé permettant de réaliser un multicapteur comportant au moins deux protéines différentes dont au moins une partie se situe à l'interface polymère conducteur milieu réactionnel, ce multicapteur étant obtenu par copolymérisation d'un monomère polymérisable et de protéines sur lesquelles peuvent être greffés un monomère identique ou différent et susceptible de copolymériser.

Ainsi, le procédé de la présente invention peut avantageusement être utilisé pour fabriquer un capteur ou un multicapteur, par exemple une biopuce, car il permet de s'affranchir des inconvénients précités de l'art antérieur. Les capteurs fabriqués fournissant des résultats d'analyse d'échantillon précis, sensibles et reproductibles.

Selon l'invention, la protéine peut être choisie par exemple dans le groupe constitué d'une enzyme, d'un anticorps, d'un antigène, d'une hormone, un récepteur, etc. ce qui donne à la présente invention un large domaine d'application.

D'autres avantages apparaîtront encore à l'homme du métier à la lecture des exemples qui suivent donnés pour illustrer la demande, et non la limiter, en référence aux figures annexées.

### Brève description des figures

- Les figures 1 à 4 représentent schématiquement les techniques de l'art antérieur, et les résultats auxquels elles conduisent (à droite).
- La figure 5 représente schématiquement le procédé de la présente invention et le résultat auquel elle conduit.
- La figure 6 représente schématiquement le dispositif utilisé pour la mise en oeuvre du procédé de l'invention.
- Les figure 7 a), b), c) et d) représentent des micrographies de la mesure des intensités de fluorescence de différents conjugués streptavidine-biotine.
- Les figures 8a) et 8b) représentent respectivement une micrographie de la mesure d'intensités de fluorescence de différents conjugués streptavidine-biotine, et un schéma des tâches (« spots ») obtenues sur la micrographie.
- La figure 9 est une représentation graphique des résultats d'essais de résonance plasmonique de surface (SPR) avec des anticorps.
- La figure 10 représente la cinétique de reconnaissance de plots constitués d'immunoglobulines G (Ig G) de lapin et d'HBT 10 (anticorps anti hCG) fixés sur un polymère conducteur selon le procédé de l'invention.
- La figure 11 est une micrographie d'intensités de fluorescence mesurées en fonction de l'épaisseur du film de polypyrrole, obtenue dans les conditions opératoires décrites dans l'exemple 7.

### EXEMPLES

### Exemple 1 : Préparation d'esters activés du pyrrole

### A) SYNTHESE DE PYRROLE SULFO N-HYDROXY-SULFOSUCCINIMIDE (SULFO NHS)

Dans un ballon de 25 ml, on ajoute dans l'ordre : de l'acide 11-(1-pyrrolyl)undécan-1-oïque (2 mmoles ; 503 mg), du N-hydroxy-sulfosuccinimide (2 mmoles ; 434 mg) et de la dicyclohexylcarbodiimide (DCC) (2,4 mmoles ; 495 mg). On ajoute ensuite 10 ml de diméthylformamide (DMF). On obtient un mélange trouble qui est placé sous agitation magnétique une nuit.

Le mélange réactionnel est ensuite filtré sous vide. Le précipité de dicyclohexylurée (DCU), formé lors de la réaction, est éliminé. Le filtrat contenant le produit est évaporé à l'évaporateur rotatif.

Le produit obtenu est repris dans 25 ml d'eau. Cette solution est lavée par trois fois 50 ml de dichlorométhane, pour éliminer la DCC résiduelle. L'eau est ensuite éliminée au Speedvac (marque déposée) pendant une nuit.

Le rendement de la réaction est de 65%.

### B) SYNTHESE DE PYRROLE MALEIMIDE

### Préparation de l'ester activé du maléimide

On met sous agitation magnétique pendant une nuit, de l'acide maléimide (3,7 g ; 17,5 mmoles), du NHS (2 g ; 17,5 mole), du DCC (3,6 g ; 17,5 mmoles), du DMF(90 ml). La réaction chimique du couplage du maléimide avec le pyrrole est représentée ci-dessous.

Le mélange obtenu est filtré pour éliminer la DCU. Le filtrat est ensuite concentré.

On obtient une poudre blanche. Le produit a été utilisé tel quel, sans purification supplémentaire.

### Couplage du maléimides avec le pyrrole:

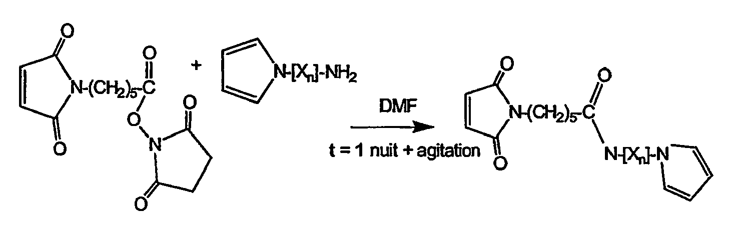

X étant un groupe -CH₂, n étant un nombre entier tel que 1 ≤ n ≤ 20.

Par exemple, [X]ₙ peut être tel que le maléimide pyrrole qui est défini ci-dessous dans les points i), ii) et iii).

### i) Maléimide-[2]-pyrrole :

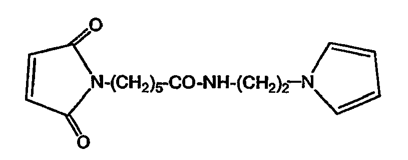

Le mélange d'ester-activé du maléimide (924 mg ; 3 mmoles), de N-aminoéthylpyrrole (330 mg ; 3 mmoles) et de 15 ml de DMF est mis sous agitation pendant une nuit. Une chromatographie sur couche mince (CCM) permet de vérifier la fin de la réaction.

Après évaporation du DMF, le produit est purifié par chromatographie sur colonne de silice. L'élution commencée avec du dichlorométhane pur, est poursuivie avec un gradient croissant de MeOH (élution du produit : 98%-2%).

La masse obtenue est de 0,4 g. Le rendement final de la réaction est donc de 45%.

### ii) Maléimide-[6]-pyrrole :

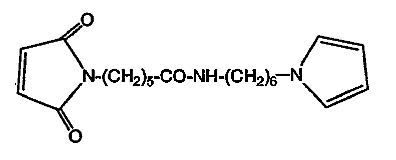

La réaction se fait sous agitation magnétique pendant une nuit après mélange dans du DMF (15 m1), de l'ester activé du maléimide (0,924 g; 3 mmoles) et du N-aminohexylpyrrole (0,498 g ; 3 mmoles). L'élution sur la colonne de silice se fait avec le mélange CH₂Cl₂/MeOH (97%-3%).

Avec une masse de 0,5 g, on trouve un rendement de cette réaction de 46,4%. Le produit obtenu est un liquide brun.

### iii) Pyrrole- [13]-maléimide :

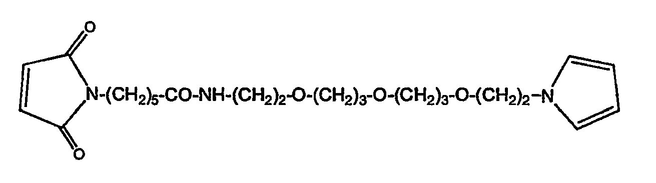

Le protocole étant identique à celui ci-dessus, on mélange de l'ester-activé du maléimide (1,14 g ; 3,70 mmoles), du pyrrole-[13]-amine (1 g ; 3,70 mmoles) dans 20 ml de DMF. L'élution sur colonne de silice se fait avec le mélange CH₂Cl₂/MeOH (97%-3%). La masse du produit obtenu (liquide brun) est de l g, soit un rendement final de 58%.

### Exemple 2 : Couplages sur la streptavidine

### A) COUPLAGE DE LA STREPTAVIDINE EN UTILISANT DE NHS-PYRROLE

Deux types de NHS-pyrroles fabriqués comme dans l'exemple 1 ci-dessus sont utilisés en comparaison : le pyrrolyl caproate de NHS, et le pyrrolylundécanoate de sulfo NHS.

On travaille avec un rapport molaire initial (RMI) égal à 40, soit 40 NHS-pyrroles pour une protéine, ainsi, 5,55 nmoles de streptavidine reprises dans 300 µl de tampon de couplage sont additionnés à 222 nmoles de NHS-pyrrole (148 µl d'une solution de NH--pyrrole 1,5 mM) quel qu'il soit. La réaction de couplage est laissée 2 heures.

Trois réactions sont menées en parallèle :
- la première utilise le pyrrolyl caproate de NHS,
- la seconde le pyrolylundécanoate de sulfo NHS,et
- la dernière est un témoin ne contenant que de la streptavidine seule en solution dans du tampon de couplage.

La purification après réaction est faite en utilisant des filtres Nalgen 30K (marque de commerce), suivant le protocole du fabriquant.

375 µl de chaque échantillon sont chacun déposés sur des filtres différents, et centrifugés 15 minutes à 8000 tr/min. Puis chaque filtre est rincé trois fois avec 200 µl de tampon de « spotting », une centrifugation de 15 à 20 minutes est nécessaire entre chaque lavage.

A la fin, trois fractions de 50 µl sont récupérées :
- Streptavidine-pyrrolyl caproate de NHS
- Streptavidine-pyrolylundécanoate de sulfo NHS
- Streptavidine seule.

### B) COUPLAGE DES MALEIMIDES-PYRROLES SUR LA STREPTAVIDINE

La streptavidine ne possédant pas de fonctions SH, elles sont crées par l'intermédiaire d'un réactif bifonctionnel, le N-succinimidyl-3-[2-pyridyldithio]propionate (SPDP), qui réagit avec les fonctions amines de la protéine. Les SH de ce réactif sont ensuite libérés par le dithiotréitol, d'où l'intérêt de l'utilisation de ces réactifs. Cette technique est décrite par exemple dans Carlson, T., Drevin, M. and Axen, R. (1978) Biochem. J. 173, 723-727.

Les trois maléimides fabriqués comme dans l'exemple 1 ci-dessus sont donc :
- l'éthyl maléimide-pyrrole
- le trioxatridécanoyle maléimide
- l'hexyl maléimide-pyrrole.

### a) Création des fonctions SH libres

On fait réagir 4,2 nmoles de streptavidine avec 150 nmoles de SPDP. La réaction est laissée 40 minutes. Puis le mélange est filtré en utilisant des filtres Nalgène 30K, et rincé trois fois avec 100 µl de tampon acétate de sodium 50 mM, pH 4,5.

62,5 nmoles de dithiotréitol sont ensuite ajoutées à la solution streptavidine-SPDP (SA-SPDP) obtenue. La réaction est laissée 40 minutes. On filtre à nouveau de la même façon que précédemment, le rinçage se faisant avec une solution tampon PBS, afin de récupérer la streptavidine (SA) avec des fonctions thiols libres accessibles (SA-SH), dans un volume de 150 µl.

L'échantillon obtenu est ensuite séparé en trois fractions de 50 µl, auxquelles sont ajoutées 85 nmoles de maléimide-pyrrole.

### b) Cas de l'éthyl maléimide-pyrrole (PM = 303 g/mol)

On mélange de la SA-SH avec une solution de maléimide-pyrrole 25 nmoles/pl (3,4 µl ; 85 nmoles) préparée en reprenant 2 mg du maléimide-pyrrole dans 200 µl de DMF. La réaction dure 30 minutes.

### c) Cas du trioxatridécanoyl maléimide (M = 463 g/mol)

La réaction se passe comme celle ci-dessus en additionnant de la SA-SH avec une solution de maléimide-pyrrole à 39 nmoles/pl (2,2 µl ; 85 nmoles) préparée en reprenant 1 mg de maléimide pyrrole dans 50 µl de DMF. La réaction est laissée 30 minutes.

### d) Cas de l'hexyl maléimide-pyrrole (PM = 359 g/mol)

La SA-SH est mélangée avec une solution de maléimide-pyrrole à 57,5 nmoles/pl (1,5 µl ; 85 nmoles) préparée en reprenant 3 mg de maléimide-pyrrole dans 200 µl de DMF. La réaction se fait pendant une demi-heure.

Après la réaction, les différents mélanges réactionnels ainsi que la solution témoin (Streptavidine seule, sans réactif ajouté) sont centrifugés sur filtres Nalgen 30K et rincés avec du tampon de spotting. Enfin, tous les échantillons sont ramenés à un volume final de 50 µl, par complément avec du tampon de spotting (tampon phosphate de sodium 50nM, pH 7).

### Exemple 3 : Synthèse électrochimique de polypyrroles (« SPOTTIN6 ») : électropolymérisation

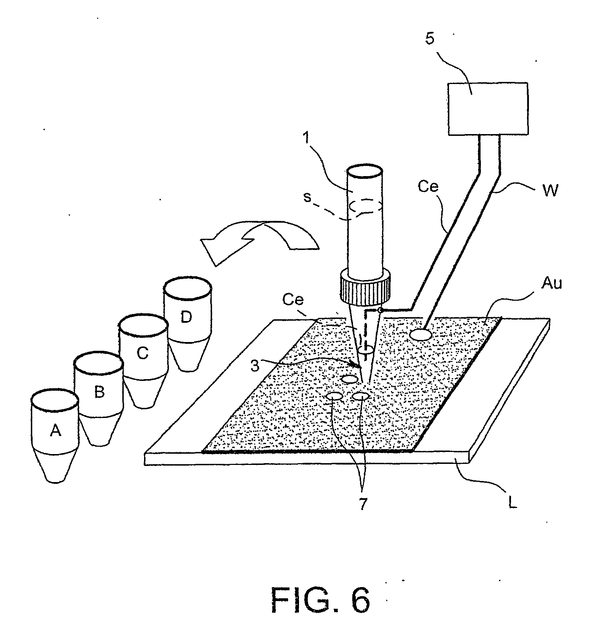

La réaction se fait en utilisant le montage de « spotting » représenté sur la figure 6 annexée, qui est décrit par ailleurs de manière détaillée dans le document P. GUEDON et al. cité ci-dessus. Sur cette figure, « A », « B » correspondent à la streptavidine couplée au polypyrrole via, respectivement, le caproate de NHS, et l'undécanoate de sulfo NHS ; « C » correspond à la streptavidine couplée au pyrrole via l'hexyl maléimide, et « D » correspond à la streptavidine seule. « L » représente une lame de verre, « Au » une couche d'or déposée sur cette lame de verre (l'ensemble est appelé ci-après « lame d'or » ; « s » la solution d'électropolymérisation ; et « W » l'électrode de travail. La référence 1 indique une pipette ; la référence 3 la pointe de la pipette ; et la référence 7 des spots de polypyrrole déposés sur la couche d'or.

Comme cela est représenté sur cette figure, la lame d'or sur laquelle est réalisée l'électropolymérisation sert d'électrode de travail (W), et un fil de platine placé dans le cône de dépôt sert de contre-électrode (Ce). Ce système électrochimique est connecté au potentiostat 5.

La copolymérisation se fait avec 20 µl d'une solution contenant 5 µl de la solution SA-maléimide-pyrrole, choisie parmi celle fabriquées dans l'exemple précédent, et 15 µl de la solution de spotting comprenant du tampon phosphate 50 mM avec un pH de 7, et du pyrrole 20 mM.

Le film est synthétisé sur la couche d'or suite à une impulsion électrique qui dure 500 ms et monte à 2,4 V (soit environ 0,9 V/ECS).

Les différents spots sont tous réalisés de la même façon, le cône étant rincé avec de l'eau entre chaque polymérisation.

### Exemple 4 : Phase de détection des protéines greffées sur les spots

Après la synthèse électrochimique de l'exemple 3, la zone où se trouvent les spots est délimitée par une barrière hydrophobe réalisée à l'aide d'un feutre de colle. Les spots sont ensuite lavés deux fois par l ml de tampon de rinçage. Puis ils sont bloqués par 100 µl du tampon de blocage, afin d'empêcher la fixation non spécifique des protéines, déposés à l'intérieur de la zone hydrophobe précédemment délimitée. Après un second rinçage identique au précédent, 100 µl d'une solution R-phycoérythrine biotinylée à 10% dans du PBS, est déposée.

La réaction est laissée 10 minutes à l'obscurité, puis un dernier lavage identique aux précédents est réalisé avant la phase de détection au microscope.

### Exemple 5 : révélation

Les échantillons déposés sur la lame d'or sont placés entre lame et lamelle et observés avec un microscope à épifluorescence équipé d'une caméra CDD sous lumière verte et à un grossissement x 1,2. Des images des spots sont prises et les intensités de fluorescence sont mesurées par le logiciel Image Pro Plus.

### A) COUPLAGES EN UTILISAIT LE PYRROLYL CAPROATE DE NHS ET LE PYRROLYL UNDECANOATE DE SULFO NHS FABRIQUES DANS L'EXEMPLE 1

Ces couplages seront, ici, mis en évidence par détection fluorescente, par l'utilisation du couple affine constitué de la biotine et de la streptavidine (SA) . L'efficacité de ces couplages sera, ainsi, jugée par leur intensité de fluorescence (IF ou niveaux de gris).

Quatre types de spots ont été réalisés selon le schéma ci-dessous :
- MR = milieu réactionnel : tampon phosphate 50 mM, pH 7, et pyrrole 20 mM :
   IF = 10 :
- A = SA couplée au pyrrole par le pyrrolyl caproate de NHS :
   IF = 200 ;
- B = SA couplée au pyrrole par le pyrrolyl undécanoate de sulfo NHS :
   IF = 240 ;
- T = témoin : SA seule + MR :
   IF = 80 ;
avec IF = intensité de fluorescence.
Noir = bruit de fond

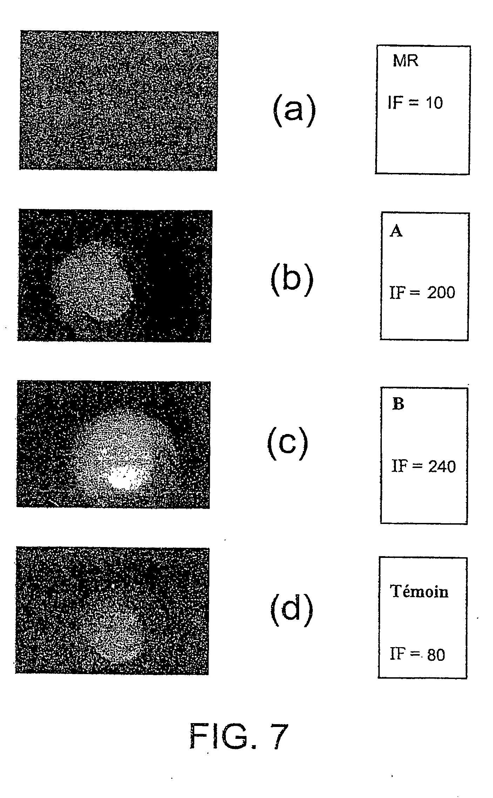

Il sont représentés dans cet ordre, respectivement sur les figures 7 a), b), c) et d) annexées.

Le spot MR contrôle l'adsorption non spécifique à la surface du film de polypyrrole. Conformément à notre attente, il présente peu de fluorescence.

Le spot Témoin, correspond à la streptavidine non couplée à du pyrrole. Elle est donc adsorbée sur le polypyrrole. Ce spot montre une intensité de fluorescence nettement inférieure à celles des échantillons SA-pyrrole, A et B. Ceci démontre que la présence du pyrrole favorise l'accrochage de la streptavidine à la surface de la plaque d'or.

Les spots A et B correspondent à la streptavidine couplée au polypyrrole via, respectivement, le caproate de NHS, et l'undécanoate de sulfo NHS. En comparant les intensités de fluorescence de ces deux spots, on constate que le signal obtenu est, un peu plus important quand la SA est couplée par le pyrrolyl undécanoate de sulfo NHS, que par le pyrrolyl caproate de NHS. Cette différence est très probablement due à la longueur de la chaîne constituant ce NHS ; elle permettrait le maintien de l'accessibilité des sites pour la fixation de la biotine.

Dans l'ensemble, on conclut que ce couplage, donnant de bons résultats sur la streptavidine, est possible sur d'autres types de protéines.

### B) COUPLAGES EN UTILISANT TROIS MALEIMIDES DIFFERENTS

Cinq types de spots ont été réalisés selon le schéma ci-dessous:
- MR = milieu réactionnel : tampon phosphate 50 mM, pH = 7 et pyrrole 20 mM
- T = témoin : MR + SA seul
- A = MR + SA couplée au pyrrole via le trioxatridécanoyl maléimide
- B = MR + SA couplée au pyrrole via l'éthyl maléimide
- C = MR + SA couplée au pyrrole via l'hexyl maléimide

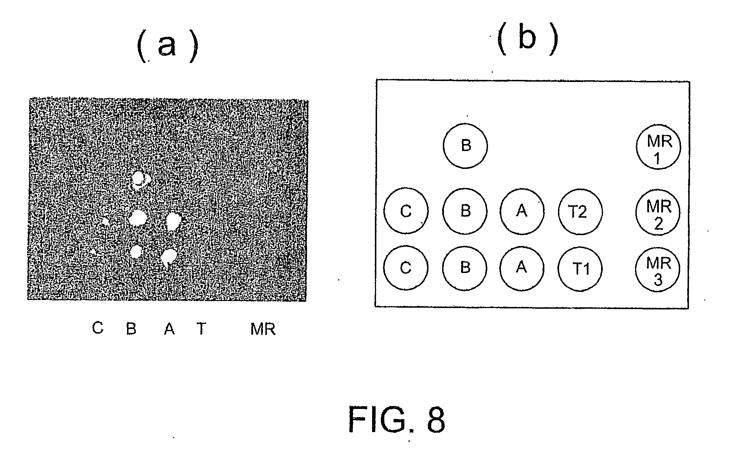

Les figures 8 (a) et (b) illustrent les mesures d'intensité de fluorescence des différents conjugués Streptavidine-pyrrole. Les spots sont disposés suivant le schéma de la figure 8 (b) . Les références sur cette figure correspondent à celles indiquée ci-dessus.

Les mesures d'intensités de fluorescence prises sur ces photos sont récapitulées dans le tableau 2 ci-dessous.

Les spots MR1, MR2 et MR3, conformément à l'attente des inventeurs, présentent peu de fluorescence, ce qui montre l'absence d'adsorption non spécifique.

Les témoins T1 et T2 correspondant à la SA non couplée au pyrrole, et donc adsorbée sur le polypyrrole, montrent une fluorescence très inférieure à celles des échantillons SA-pyrroles.

Ainsi la présence du pyrrole sur la molécule favorise, ici aussi, l'accrochage de la streptavidine.

En comparant les intensités de fluorescence de ces trois bras espaceurs, on constate qu'on a une meilleure fluorescence pour le bras, trioxatridécanoyle, suivi du bras éthyle, et enfin du bras hexyle.

**Tableau 2**

| | | |
|---|---|---|
| **Récapitulatif des intensités lumineuses des différents polymères** | | |

| **Electropolymérisation** | | **Intensité de fluorescence** |
|---|---|---|
| SA + pyrrole 1 | A1 | 960 |
| | A2 | 1025 |
| SA + pyrrole 2 | B1 | 497 |
| | B2 | 457 |
| | B3 | 437 |
| SA + pyrrole 3 | C1 | 150 |
| | C2 | 200 |
| SA seule | T1 | 96 |
| | T2 | 91 |
| MR | MR1 | 10 |
| | MR2 | 10 |
| | MR3 | 10 |

En l'état actuel des travaux, les inventeurs ne sont pas en mesure de savoir avec certitude pourquoi un bras présente un meilleur signal qu'un autre. La réponse à cette question nécessite des études et des caractérisations supplémentaires. Les axes de réflexion pourraient être, par exemple, la longueur et/ou le caractère hydrophile plus ou moins important des bras ; mais aussi l'efficacité du couplage, etc.

### Exemple 6 : Les anticorps

### A) COUPAGE DES ANTICORPS AVEC LE NUS-PYRROLE

On utilise ici le pyrrolyl caproate de NHS et trois types d'anticorps différents :
i) une Immunoglobuline G de lapin
ii) une Immunoglobuline anti hCG ("human Chorionic Gonadotropin").

### i) IgG de lapin

10 mg d'immunoglobuline G (Ig G) sont repris dans 333 µl de PBS. On travaille avec un RMI de 40. Ainsi 12,5 µl (2,5 nmoles) de cette solution ont été mélangés à 6,7 µl (100 nmoles) d'une solution de pyrrolyl caproate de NHS 15 mM dans de la DMSO. La réaction est laissée 2h30.

Les échantillons sont ensuite purifiés à l'aide de filtres Nalgen 30K, à 8000 tr/min pendant 20 minutes, et rincés trois fois avec 100 µl de tampon de spotting, une centrifugation de 20 minutes étant nécessaire entre chaque rinçage.

Enfin, les Ig G-pyrrole sont récupérées dans un volume de 50 µl de tampon de spotting.

### ii) Immunoglobuline anti hCG

On dispose de deux anticorps (Ig G) anti hCG différents, tous deux issus de la souris, l'HT 13 et le FBT 10. Le protocole suivi est le même pour chacun d'eux.

1 nmole d'anticorps est additionnée à 67 µl d'une solution de pyrrolyl caproate 15 mM dans la DMSO. On laisse agir 2h30.

Les échantillons sont filtrés, comme précédemment, à l'aide de filtres Nalgen, rincés trois fois avec 100 µl de tampon de spotting. Deux échantillons d'Ig G anti hCG-Pyrrole sont récupérés en solution dans du tampon de spotting et dans un volume de 50 µl.

### B) SYNTHESE ELECTROCHIMIQUE DU POLYPYRROLE : SPOTTING

La copolymérisation est la même pour les deux modèles considérés.

Ici, elle est réalisée avec 40 µl d'une solution contenant 20 µl de la solution Ig G-pyrrole (quelle qu'elle soit) et 20 µl de solution de spotting (tampon phosphate 20 mM, pyrrole 20mM).

### C) PHASE DE DETECTION DES ANTICORPS GREFFES SUR LES SPOTS PAR FLUORESCENCE

Dans les deux modèles suivants, les spots sont rincés deux fois avec 1 ml de tampon PBS 10 mM, BSA 1%, Tween 0,05% (p/p) entre chaque étape, et toutes les réactions sont faites à température ambiante.

### i) IgG de lapin

Après la synthèse électrochimique, les spots sont rincés puis bloqués avec 100 µl de tampon de rinçage (PBS 10 mM, BSA 1%, Tween 0,05% (p/p)) pendant 30 minutes. Après un second rinçage, 100 µl d'une solution d'anticorps anti Ig G de lapin ou d'homme biotinylé respectivement dilués au 1/10 000 et au 1/50 000, sont ajoutés. La réaction est laissée 30 minutes. Après un nouveau rinçage, 100 µl d'une solution de streptavidine R-phycoérythrine à 10% dans du tampon de rinçage sont ajoutés 10 minutes à l'obscurité. La détection de fluorescence est réalisée comme précédemment dans l'exemple 4.

### ii) Immunoglobuline anti hCG

Le protocole suivi est le même que précédemment en ce qui concerne les étapes de blocage et de rinçage. Après blocage et rinçage, 100 µl d'une solution d'hCG 300 nM dans du tampon PBS 10 mM, BSA 1%, Tween 0,05%(p/p) est déposée sur les spots. La réaction est laissée 30 minutes. On rince à nouveau, puis 100 µl d'une solution d'anticorps anti hCG différent de celui fixé sur la plaque, et provenant du lapin, dilué au 1/2 000 sont ajoutés. On laisse agir 30 minutes. Un nouveau rinçage est effectué, puis 100 µl d'une solution d'anti Ig G de lapin biotinylé diluée au 1/10 000 sont ajoutés.

La réaction est laissée 30 minutes. Après rinçage, la révélation est réalisée comme précédemment.

### D) EXPERIENCES DE RESONANCE PLASMONIQUE DE SURFACE (SPR) AVEC LES ANTICORPS

Afin de pouvoir analyser la fonctionnalité des plots, les inventeurs se sont placés à incidence fixe pour mesurer les cinétiques de variation de la réflectivité en fonction du temps.

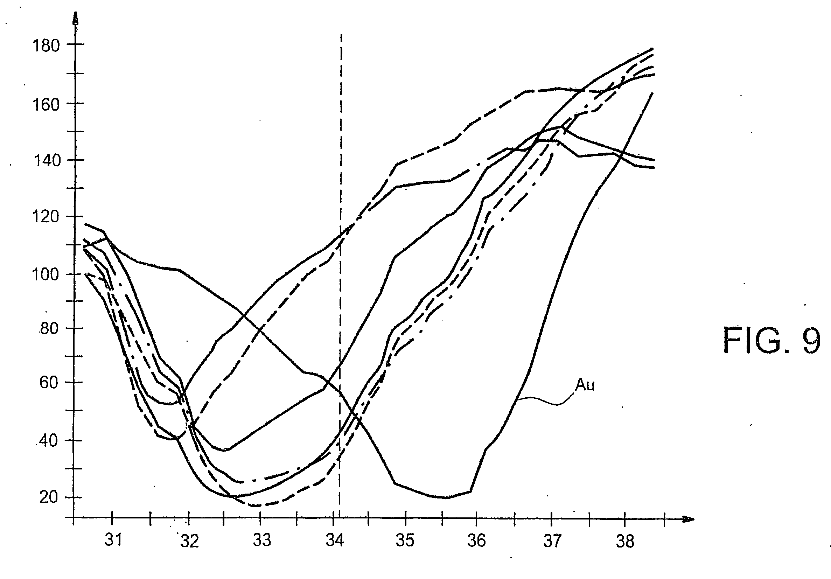

Le détail de l'instrumentation utilisée est donné dans le document P. GUEDON et al. cité ci-dessus. La figure 9 est une représentation graphique du plasmon en tampon obtenu.

Les inventeurs se sont donc placés vers 34,1°, pour être dans le flanc droit du pic d'absorption de la courbe de réflectivité.

Le protocole était le suivant :
- passage du bloquant, solution contenant du PBS 10 mM, de la BSA 1% et du tween 0,05%, destiné à limiter les interactions non spécifiques par la suite,
- mesure et enregistrement de la réflectivité en fonction de l'angle d'incidence en milieu bloquant qui sert de tampon,
- passage de l'anti-IgG lapin biotinylée, diluée au 1/100,
- retour en bloquant afin de mesurer la différence de niveau dans le même milieu avant et après la prise d'anticorps,
- passage de l'hCG diluée au 1/100,
- retour en bloquant afin de mesurer la différence de niveau dans le même milieu avant et après la prise d'anticorps,
- passage de l'IgG anti-hCG issue du lapin dilué au 1/2 000,
- retour en bloquant afin de mesurer la différence de niveau dans le même milieu avant et après la prise d'anticorps,
- passage de l'anti-IgG lapin biotinylée, diluée au 1/100,
- retour en bloquant afin de mesurer la différence de niveau dans le même milieu avant et après la prise d'anticorps,
- passage du tampon de régénération, solution contenant de la Glycine 0,1 M et de l'acide chlorhydrique (pH = 2,3),
- retour en bloquant afin de comparer le niveau initial en bloquant lors du plasmon et le niveau final.

Cette plaque est en premier lieu révélée par des anti Ig G de lapin biotinylés puis par de l'hCG, des anti hCG issus du lapin, et enfin des anti Ig G de lapin biotinylés. L'expérience dure 5h20 min.

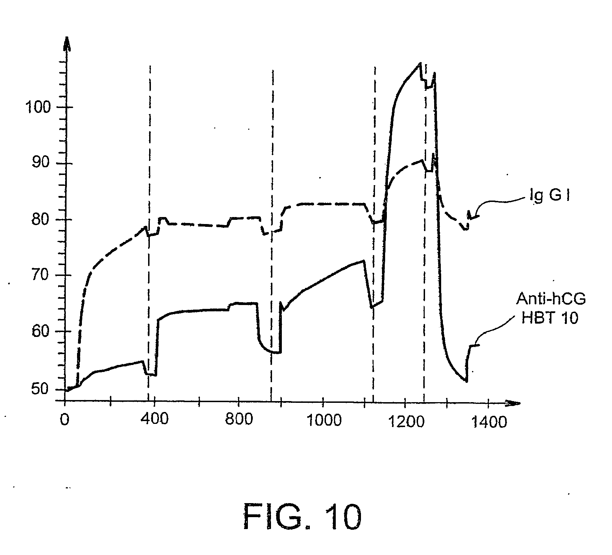

La figure 10 annexée est une représentation graphique de la cinétique des dépôts de la plaque : 2 dépôts constitués d'Ig G de lapin (IgG 1) et d'HBT 10 (anticorps anti hCG).

Cette expérience démontre que l'empilement des différentes protéines se fait comme voulu. On constate ainsi bien les différents paliers correspondants à l'attachement des protéines injectées sur le ou les dépôts. Le dépôt constitué d'Ig G de lapin ne prend que des anti Ig G de lapin, et pas d'hCG. Le dépôt constitué d'HBT 10 fixe bien l'hCG, ceci prouvant qu'il n'y a pas de réaction de croisement entre les deux types d'anticorps de lapin, à savoir, les Ig G de lapins fixés sur la plaque et les anti hCG issus du lapin.

### Exemple 7 : Influence de l'épaisseur de dépôt de polymère sur la capacité de reconnaissance d'une protéine greffée sur/dans ce polymère.

Un anticorps anti IgG de lapin est greffé dans le polymère selon le procédé donné dans l'exemple 6.

Les dépôts sont réalisés à partir du même milieu réactionnel (tampon phosphate de sodium 50 mM pH 7, pyrrole 20 mM IgG). Les dépôts, réalisés en doublet pour A à F et en triplet pour G sont réalisés par électrospotting durant des temps variables générant ainsi des polymères d'épaisseurs variables. Les charges de synthèse sont mesurées et l'épaisseur du polymère peut ainsi être évaluée.

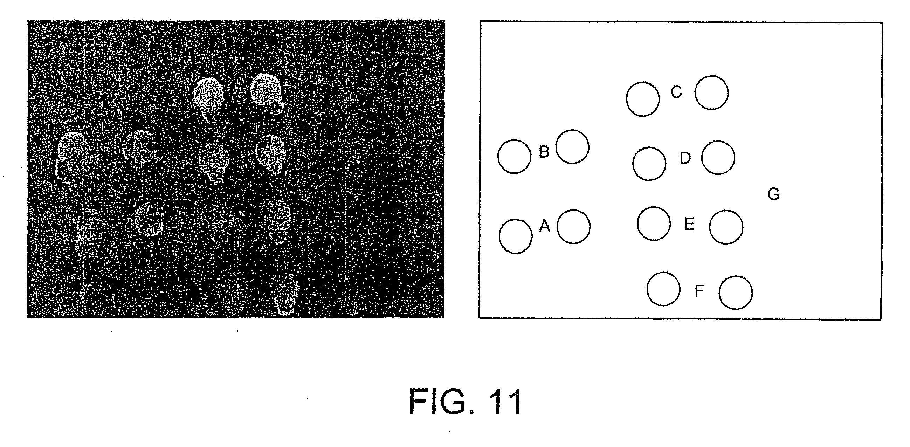

La biopuce fabriquée est ensuite traitée avec des antiIgG de lapin biotinylées puis la reconnaissance est détectée grâce à la streptavidine-phycoérythrine (voir exemple 6). L'image obtenue est fournie sur les figures 11 (a) et (b) annexées et les résultats quantitatifs (Intensités de Fluorescence) sont donnés dans le 3 tableau ci-dessous. La figure 11 (b) est une représentation schématique de la photographie 11 (a) permettent de mettre en évidence les dépôts.

Les résultats montrent que lorsque le polymère est trop épais, la reconnaissance avec l'anti IgG de lapin (grosse molécule) se fait difficilement. On arrive dans le cas des polymères épais à un système d'emprisonnement comme celui décrit par Wolowaczs et al. dans Anal. Chem. 1992, 64, 1541-1545. Dans le cas de la fluorescence, l'épaisseur optimale est de l'ordre de 10 nm, ce qui correspond a peu près au diamètre de la molécule immobilisée. Elle ne peut donc pas être simplement emprisonnée dans le polymère ce qui implique une fixation covalente. Cette même épaisseur de polymère (inférieure à 10nm) permet par ailleurs d'optimiser la détection par résonance plasmonique (P. GUEDON et al, Anal. Chem. 2000 72, (24), 6003-6009).

**Tableau 3**

| plots | Durée de synthèse (ms) | Charge (µC) | Charge (µC/mm²) | Epaisseur (nm) | IF (UA) |
|---|---|---|---|---|---|
| A | 250 | 10 | 25 | 5 | 90 |
| B | 500 | 15 | 37 | 7,2 | 102 |
| C | 1000 | 20 | 50 | 10 | 160 |
| D* | 2000 | 37 | 90 | 18 | 140 |
| E* | 4000 | 58 | 142 | 28 | 90 |
| F* | 8000 | 115 | 287 | 56 | 80 |
| G* | 16000 | 205 | 512 | 102 | 30 |

| | | | | | |
|---|---|---|---|---|---|
| *comparatif | | | | | |

### Conclusion

L'épaisseur du polymère joue donc un rôle clé dans la capacité de fixation et surtout dans la capacité de reconnaissance de protéines encombrées. On peut donc comparer les épaisseur ou les densités de charge d'électropolymérisation décrits dans la littérature : Wolowacz et al propose des films incorporant de la Glucose oxydase, les densités de polymérisation sont de l'ordre de 1, 4 mC/mm² (soit une épaisseur de film d'environ 280 nm). Dans ce cas, le capteur fonctionne car il permet, de par la porosité du polymère, la diffusion d'un petit analyte, le glucose. Plus récemment, Sadik et al dans le document précité proposent un capteur immunologique sur le modèle de la sérum albumine humaine (HSA), l'épaisseur du film de polypyrrole utilisé pour l'emprisonnement de la protéine est au minimum de 350 nm soit une densité de charge de polymérisation de 1,5 mC/mm².

Aucun art antérieur n'a décrit ou suggéré que l'épaisseur du polymère peut avoir une influence sur la réponse biologique d'un biocapteur. Les inventeurs sont donc les premiers à fournir un procédé qui prend en compte ce paramètre permettant, de manière inattendue, de résoudre les problèmes précités de l'art antérieur.

## Revendications

1. Procédé de fixation d'une protéine sur un support conducteur au moyen d'un polymère du pyrrole comprenant les étapes suivantes :
- couplage de la protéine à fixer avec un monomère du pyrrole de manière à obtenir une première solution d'un composé de couplage protéine-pyrrole,
- préparation d'une deuxième solution du monomère du pyrrole non couplé à la protéine,
- mélange de ladite première solution avec ladite deuxième solution pour obtenir une solution d'électropolymérisation,
- électropolymérisation du pyrrole et de la protéine couplée au pyrrole sur au moins une zone déterminée du support conducteur à partir de ladite solution d'électropolymérisation, ladite électropolymérisation étant réalisée en délivrant sur ladite zone une quantité de courant pour obtenir un polymère ayant une épaisseur au plus égale à 10 nm.

2. Procédé selon la revendication 1, dans lequel la, au moins une, zone conductrice sur laquelle l'électropolymérisation est réalisée est, au moins un plot d'un support de biocapteur.

3. Procédé selon la revendication 1, dans lequel le couplage de la protéine à fixer avec du pyrrole est réalisé au moyen d'une activation du pyrrole suivie d'un couplage du pyrrole activé à la protéine à fixer.

4. Procédé selon la revendication 3, dans lequel l'activation du pyrrole est réalisée au moyen de N-hydroxy-sulfosuccinimide ou de maléimide.

5. Procédé selon la revendication 3, dans lequel le composé de couplage protéine-pyrrole est choisi parmi les composés suivants : 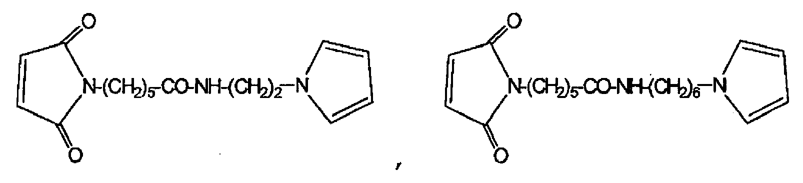 et

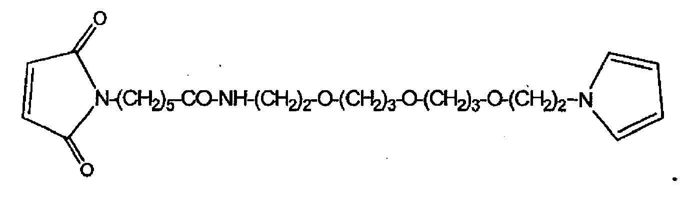

6. Procédé selon la revendication 1, dans lequel deux protéines sont fixées sur le polymère du pyrrole, successivement et sur deux zones différentes déterminées du support conducteur.

7. Procédé selon la revendication 1, dans lequel la protéine est choisie dans le groupe constitué d'une enzyme, d'un anticorps, d'un antigène, d'une hormone, d'un récepteur.

8. Utilisation d'un procédé selon l'une quelconque des revendications 1 à 7 précédentes pour fabriquer un monocapteur ou un multicapteur.

9. Utilisation d'un procédé selon l'une quelconque des revendications 1 à 7 précédentes pour fabriquer une biopuce.

## Claims

1. Method for attaching a protein to a conductive support by means of a pyrrole polymer, comprising the following steps:
- coupling of the protein to be attached with a pyrrole monomer so as to obtain a first solution of a protein-pyrrole coupling compound,
- preparation of a second solution of the pyrrole monomer not coupled to the protein,
- mixing of said first solution with said second solution so as to obtain an electropolymerization solution,
electropolymerization of the pyrrole and of the protein coupled to the pyrrole on at least one given area of the conductive support using said electropolymerization solution, said electropolymerization being carried out by delivering onto said area an amount of current to obtain a polymer presenting a thickness not exceeding 10 nm.

2. Method according to Claim 1, in which the at least one conductive area on which the electropolymerization is carried out is at least one block of a biosensor support.

3. Method according to Claim 1, in which the coupling of the protein to be attached with pyrrole is carried out by means of activation of the pyrrole followed by coupling of the activated pyrrole to the protein to be attached.

4. Method according to Claim 3, in which the activation of the pyrrole is carried out by means of N-hydroxysulphosuccinimide or of maleimide.

5. Method according to Claim 3, in which the protein-pyrrole coupling compound is chosen from the following compounds: 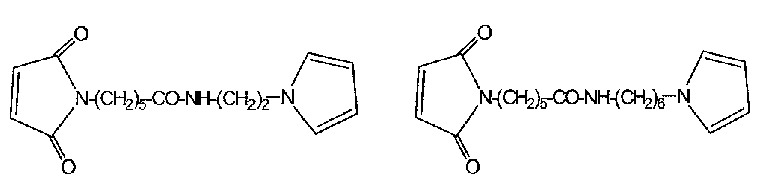 and

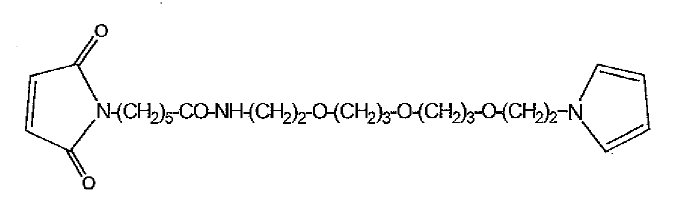

6. Method according to Claim 1, in which two proteins are attached to the pyrrole polymer, successively and on two different given areas of the conductive support.

7. Method according to Claim 1, in which the protein is chosen from the group consisting of an enzyme, an antibody, an antigen, a hormone and a receptor.

8. Use of a method according to any one of the preceding Claims 1 to 7, for producing a monosensor or a multisensor.

9. Use of a method according to any one of the preceding Claims 1 to 7, for producing a biochip.

## Patentansprüche

1. Verfahren zur Fixierung eines Proteins auf einem leitfähigen Träger mittels eines Pyrrolpolymers, die folgenden Schritte umfassend:
- Kopplung des zu fixierenden Proteins mit einem Pyrollmonomer, um eine erste Lösung einer Protein-Pyrrol-Kopplungsverbindung zu erhalten,
- Herstellung einer zweiten Lösung des nicht an das Protein gekoppelten Pyrrolmonomers,
- Mischung der genannten ersten Lösung mit der genannten zweiten Lösung, um eine Elektropolymerisationslösung zu erhalten,
- Elektropolymerisation des Pyrrols und des an das Pyrrol gekoppelten Proteins in wenigstens einer bestimmten Zone des leitfähigen Trägers aus der genannten Elektropolyerisationslösung, wobei die genannte Elektropolymerisation realisiert wird, indem in die genannte Zone eine Strommenge geliefert wird, um ein Polymer mit einer Dicke von höchstens gleich 10 nm zu erhalten.

2. Verfahren nach Anspruch 1, bei dem die wenigstens eine leitfähige Zone, in der die Elektropolymerisation realisiert wird, wenigstens eine Kontaktstelle eines Biosensorträgers ist.

3. Verfahren nach Anspruch 1, bei dem die Kopplung des zu fixierenden Proteins mit Pyroll realisiert wird mittels einer Aktivierung des Pyrolls, gefolgt von einer Kopplung des aktivierten Pyrolls an das zu fixierende Protein.

4. Verfahren nach Anspruch 3, bei dem die Aktivierung des Pyrrols realisiert wird mittels N-Hydroxy-Sulfosuccinimid oder Maleimid.

5. Verfahren nach Anspruch 3, bei dem die Protein-Pyrrol-Kopplungsverbindung ausgewählt wird unter den folgenden Verbindungen: 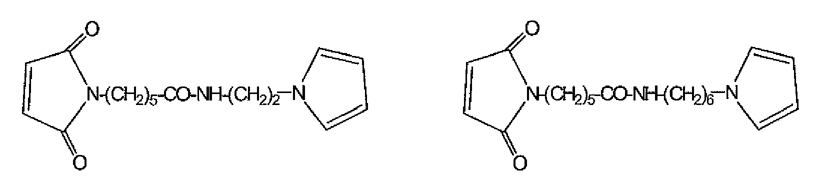 und

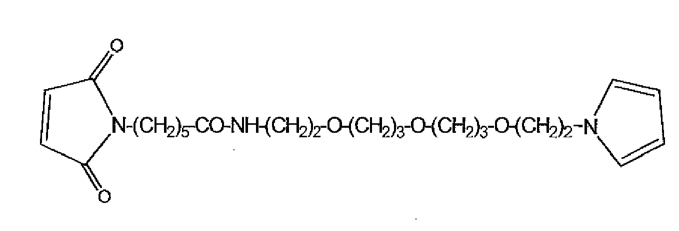

6. Verfahren nach Anspruch 1, bei dem zwei Proteine an dem Pyrrolpolymer fixiert werden, sukzessiv und in zwei bestimmten unterschiedlichen Zonen des leitfähigen Trägers.

7. Verfahren nach Anspruch 1, bei dem das Protein ausgewählt wird aus der durch ein Enzym, einen Antikörper, ein Antigen, ein Hormon, einen Rezeptor gebildeten Gruppe.

8. Anwendung eines Verfahrens nach einem der vorhergehenden Ansprüche 1 bis 7 zur Herstellung eines Monosensors oder eines Multisensors.

9. Anwendung eines Verfahrens nach einem der vorhergehenden Ansprüche 1 bis 7 zur Herstellung eines Biochips.
